# EUROPÄISCHE PATENTANMELDUNG

(11) **EP 2 336 112 A1**
(43) Veröffentlichungstag der Anmeldung: **22.06.2011**
(21) Anmeldenummer: 10186481.7
(22) Anmeldetag: 05.10.2010
(51) Int. Cl.: C07D 225/02

(54) **Verfahren zur Aufarbeitung eines Laurinlactam enthaltenen Stoffstroms für die Rückgewinnung aller enthaltene Wertstoffkomponenten durch Kombination von Kristallisation mit nachgeschalteter Destillation**

(30) Priorität: 20.11.2009 DE 102009046910
(71) Anmelder: Evonik Degussa GmbH, 45128 Essen (DE)
(72) Erfinder: Hengstermann, Axel, 48308 Senden (DE); Meier, Ralf, 44265 Dortmund (DE); Demicoli, Daniel, 45131 Essen (DE); Roos, Martin, 45721 Haltern am See (DE); Günzel, Bernd, 46514 Schermbeck (DE); Hübner, Frank, 64372 Ober-Ramstadt (DE)

(57) **Zusammenfassung**

Die vorliegende Erfindung betrifft ein Verfahren zur Aufreinigung von Laurinlactam mittels integrierter Verschaltung von Destillation und Kristallisation. Die Kristallisation kann hierbei als Lösungs- oder Schmelzkristallisation ausgeführt sein. Der aufzuarbeitende Stoffstrom enthält neben der Zielkomponente Laurinlactam mindestens eine von Laurinlactam verschieden leichter oder schwerer siedende Komponente.

## Beschreibung

Die vorliegende Erfindung betrifft ein Verfahren zur Aufreinigung von Laurinlactam mittels integrierter Verschaltung von Destillation und Kristallisation. Die Kristallisation kann hierbei als Lösungs- oder Schmelzkristallisation ausgeführt sein. Der aufzuarbeitende Stoffstrom enthält neben der Zielkomponente Laurinlactam mindestens eine von Laurinlactam verschieden leichter oder schwerer siedende Komponente. Das bei der Kristallisation abgetrennte Laurinlactam weist bereits eine Reinheit von >99% auf.

Die Aufreinigung von Laurinlactam erfolgt üblicherweise über eine mehrstufige Destillation, in der Nebenkomponenten mit höherem und niedrigerem Siedepunkt von Laurinlactam abgetrennt werden. Zur Destillation muss aufgrund des hohen Siedepunktes von Laurinlactam ein Vakuum angelegt werden. Die thermische Belastung des Gemisches in jedem Verdampfungsschritt führt zum Teil zur thermischen Zersetzung des Wertstoffs. Dies führt zur Reduzierung der Gesamtausbeute.

Die Aufgabe des erfindungsgemäßen Verfahrens besteht darin, ein Aufarbeitungsverfahren, wie in den Ansprüchen beschrieben, zu erarbeiten, dass folgende Randbedingungen erfüllt:
- Frühzeitige Abtrennung von Laurinlactam im Aufarbeitungsprozess
- Reduzierung der thermischen Produktbelastung durch Anzahlreduzierung der notwendigen thermischen Trennschritte
- Reduzierung der thermischen Belastung durch Absenkung der notwenigen Prozesstemperatur
- Rückgewinnung aller im Stoffstrom enthaltenen Wertstoffe, wie Edukte und / oder Lösungsmittel
- Abtrennung und Ausschleusung der von Zielprodukt / Edukten und Lösungsmitteln verschiedene Komponenten

Gelöst wurde die Aufgabe durch ein Verfahren gemäß den Ansprüchen, die dabei als Teil der Beschreibung zu sehen sind.

Beansprucht wird ein Verfahren zur Aufarbeitung eines Laurinlactam enthaltenen Stoffstroms für die Rückgewinnung aller enthaltenen Komponenten, dadurch gekennzeichnet, dass der aus der Synthese kommende Stoffstrom, der Laurinlactam und weitere Komponenten enthält, zunächst abgekühlt wird, so dass selektiv durch Lösungskühlungskristallisation nur die Löslichkeitsgrenze von Laurinlactam überschritten und Laurinlactam gezielt auskristallisiert und in einer nachgeschalteten Fest-Flüssig-Trennung von der Mutterlauge abgetrennt wird und die Mutterlauge anschließend einer mehrstufigen Destillationssequenz zugeführt wird.

Die Kristallisation kann als Lösungskristallisation, als Lösungskühlungskristallisation oder Flashkühlungskristallisation ausgeführt werden.

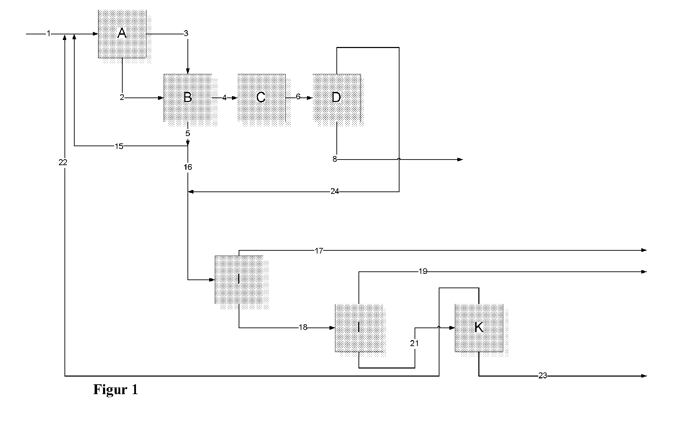

Es wurde für einen aus Laurinlactam und hiervon verschiedenen Komponenten bestehenden Stoffstrom folgende Aufarbeitungssequenz gemäß den Ansprüchen gefunden. Beispielhaft wird das Verfahren anhand des angehangenen Fließbilds (Figur 1) mit folgender Stoffstromzusammensetzung beschrieben:
LB - Leichtsieder
LM -Lösungsmittel
MB1 -Mittelsiederfraktion 1
CDON - Cyclododecanon als Reaktand der Synthese
MB2 - Mittelsiederfraktion 2
HB - Schwersieder
LL-Laurinlactam

Das Fließbild (Figur 1) ist nur beispielhaft und soll das Verfahren nicht einschränken. Der aus einer Synthesesequenz kommende und aus Laurinlactam, Lösungsmittel, CDON und hiervon verschiedenen Komponenten bestehende Stoffstrom 1 wird zusammen mit der Mutterlaugenrückführung 15, bestehend aus Laurinlactam Lösungsmittel, CDON und hiervon verschiedenen Komponenten, und dem Destillat 22 eines Verdampfungsapparates K, überwiegend Laurinlactam enthaltend, mit einer Temperatur von mindestens 75 °C, vorzugsweise mindestens 80 °C und besonders bevorzugt mindestens 85°C einer Flashkühlungskristallisation A zugeführt. Das in der Flashkühlungskristallisation A reduzierte Vakuum stellt ein entsprechendes Siedegleichgewicht ein. Die aus der Temperaturdifferenz zwischen Eingangsstrom und Siedetemperatur erhaltene Wärmemenge Q führt zur Verdampfung der leichtflüchtigen Bestandteile und zur Temperaturabsenkung entsprechend des Siedegleichgewichts. Durch die Überlagerung der Effekte wird die Löslichkeitsgrenze von Laurinlactam überschritten und Laurinlactam kristallisiert aus. Am Kristallerausgang wird eine Temperatur von maximal 70 °C, vorzugsweise darunter, bevorzugt wird eine Temperatur von ca. 65°C eingestellt. Die Suspension 2 wird aus der Flashkühlungskristallisation A zur Fest-Flüssig-Trennung B gefahren. Hierin wird die Suspension in Mutterlauge 5 und vorwiegend Laurinlactam 4 enthaltenen Feststoff getrennt. Der feuchte Feststoff 4 wird mit kondensiertem, vorwiegend Lösungsmittel enthaltenden, Destillat 3 von den anhaftenden Nebenkomponenten befreit. Die anfallende Mutterlauge und die beladene Waschlauge werden zu Strom 5 vereinigt. Ein Teilstrom 15 wird zur Ausbeutesteigerung und Einstellung des Feststoffgehalts wieder in die Flashkühlungskristallisation A eingefahren.

Der gewaschene feuchte Feststoff 4 wird einer Aufschmelzeinheit C zugeführt. Die Aufschmelzeinheit C wird auf einer Temperatur oberhalb des Schmelzpunkts von Laurinlactam betrieben. Der aufgeschmolzene Strom 6 wird einer Flashstufe D zugeführt. Am Kopf der Flashstufe wird ein Brüdenstrom 24 abgenommen und einer destillativen Aufarbeitungssequenz, die an dieser Stelle nicht weiter beschrieben wird, zugeführt. Der Sumpfstrom 8 bestehend aus spezifikationsgerechtem Laurinlactam wird abgenommen und verlässt das Verfahren.

Die Ströme 16 und 24 werden der destillativeb Lösungsmittel- und Nebenkomponentenabtrennung bestehend aus den Sequenzen I, J und K zugeführt.

### Detailbeschreibung

Die Kristallisation A wird als sog. Flashkühlungskristallisation ausgeführt. Das heißt, dass die im Feedstrom 1+15+22 enthaltene Wärmemenge Q zur Teilverdampfung des Lösungsmittels 3 eingesetzt wird. Damit es zur Verdampfung des Lösemittels kommt, wird entsprechend des Dampfdrucks des Feedstroms ein entsprechender Druck und damit eine entsprechende Kristallerinnentemperatur eingestellt. Durch die gezielte Abdampfung des Lösemittels 3 und der Abkühlung der Lösung wird die Löslichkeitsgrenze von Laurinlactam überschritten und kristallisiert aus. Durch eine effiziente Verschaltung kann die zur Kristallisation notwendige Übersättigung auf einem moderaten Niveau eingestellt werden. Die Siedekühlung erlaubt es, im Kristaller komplett auf einen direkten Wärmeaustausch zu verzichten, so dass Verkrustungsneigungen von wärmeübertragenden Flächen stark reduziert werden können. Die Abfuhr der entsprechenden Wärmemenge erfolgt über den Kopfkondensator.

Der bei der Kristallisation im Strom 2 anfallende Feststoff wird mittels einer nachgeschalteten Fest-Flüssig-Trennung B von der Mutterlauge 5 abgetrennt. Zur Entfernung der anhaftenden Mutterlauge von der Feststoffoberfläche wird dieser mit einer entsprechenden Waschflüssigkeit gewaschen. Zur Wäsche des Feststoffs konnten mehrere Waschflüssigkeiten identifiziert werden, die eine geringe Löslichkeit des Laurinlactams aufweisen. Idealerweise sollte eine Waschflüssigkeit eingesetzt werden, die bereits im Prozess vorhanden, die anhaftenden Komponenten löst und entsprechend aufgearbeitet werden kann. Hierbei konnte unter Anderem das im Kristallisationsschritt A abgetrennte Lösungsmittel 3 als ein mögliches Waschmittel identifiziert werden.

Das feuchte Kristallgut 4 muss in einem anschließenden Trocknungsschritt C und D vom anhaftenden Waschmittel befreit werden. Es hat sich gezeigt, dass es apparativ vorteilhaft ist, Laurinlactam in einem Aufschmelzer C aufzuschmelzen und über eine gewöhnliche Flashverdampfung D vom Lösungsmittel 24 abzutrennen. Hierbei ist aus Betriebserfahrungen bekannt, dass ein Mitriss von Laurinlactam nicht verhindert werden kann. Der Mitriss führt zur Belegung von installierten Wärmetauscherflächen und erfordert daher ein dem Fachmann bekanntes spezielles Kondensationssystem für Strom 24. Auf das Kondensattionssystem wird an dieser Stelle nicht eingegangen, so dass der Strom 24 das Verfahren verlässt.

Die installierte Mittelsiederabtrennung I und J kann integriert als Trennwandkolonne ausgeführt, deren Vorteile dem Fachmann hinreichend bekannt sind. Am Kopf der Kolonne I/J wird der Mittelsieder 17 mit einem niedrigeren Siedepunkt als das enthaltene Wertprodukt CDON abgenommen. Durch die installierte Trennwand innerhalb der Kolonne I/J kann am Sumpf der Kolonne restliches Laurinlactam mit den enthaltenen Schwersiedern über Strom 21 abgenommen werden. Das enthaltene CDON und die Mittelsieder mit einem höheren Siedepunkt als CDON können jeweils innerhalb der zulässigen Spezifikationsgrenzen über zwei Seitenabzüge 19 auf der Feed abgewandten Seite der Trennwandkolonne abgenommen werden.

Das an Laurinlactam und Schwersieder reiche Sumpfprodukt 21 wird von der MittelsiederDestillation I/J zur Abtrennung von Schwersiedern einem weiteren Aufarbeitungsschritt K zugeführt. Dieser besteht aus einem dem Fachmann hinreichend bekannten Verdampfungsapparat für hochviskose Fluide. Hierbei werden die hochsiedenden Komponenten über den Sumpfstrom 23 ausgeschleust. Die kondensierten Brüden 22, die den Großteil des ursprünglich im Sumpfprodukt der Mittelsiederdestillation I/J vorhandenen Laurinlactams enthalten, werden zur Ausbeuteerhöhung in die Aufarbeitungssequenz zurückgeführt. Die Rückführung erfolgt dabei in die Kristallisation A.

Das aufgezeigte Verfahren gemäß den Ansprüchen zur Aufarbeitung von Laurinlactam und zur Rückgewinnung der enthaltenen Wertstoffkomponenten, wie Lösungsmittel und Reaktand, zeigt folgende Vorteile gegenüber einem konventionellen Destillationsverfahren auf:
- Ein Großteil (größer 80 %, vorzugsweise größer 85 % und besonders bevorzugt größer 90%) des Wertstoffs kann über die Kristallisation nur durch Kühlung des Stoffstroms abgetrennt werden.
- Der Hauptanteil des produzierten Laurinlactams durchläuft keine heißen Trennschritte, bei denen es zur thermischen Belastung oder Zersetzung kommen kann.
- Durch Reduzierung des Schwersiederanteils (Laurinlactam) kann die Sumpftemperatur der Trennwandkolonne auf einem moderaten Temperaturniveau betrieben werden
- Die Kombination aus Kristallisation und Destillation reduziert die Anzahl der notwendigen thermischen Trennoperationen auf ein Minimum.
- Durch die geringe thermische Belastung des Laurinlactam wird die Bildung von Polymerisaten aus Laurinlactam reduziert, so dass der Einsatzstofffaktor über die Aufarbeitungssequenz verbessert wird.
- Das bei der Kristallisation abgetrennte Laurinlactam weist eine Reinheit von >99% auf.

### Ausführungsbeispiel:

In einen gerührten Leitrohrkristaller mit einer Innentemperatur von 35°C und einem Flüssigkeitsvolumen von 3,2L wird ein Laurinlactam enthaltener Stoffsrom mit 1,5kg/h bei einer Temperatur von 90°C eingefahren. Das Druckniveau des Kristaller beträgt ca. 0,05 bar abs. Der Stoffstrom enthält neben 20Gew.-% Laurinlactam (LL) und ca. 77,5Gew.-% Lösungsmittel (LM) und 2,5Gew.-% von LL und LM verschiedene Nebenkomponenten. Der zugeführte Stoffstrom wird mit einem Vordruck, der größer als der Siededruck von Lösungsmittel ist, dem Kristaller über eine Querschnittsverengung zugeführt. Sobald der Stoffstrom in das Flüssigkeitsvolumen eintritt, verdampft eine dem äquienthalphische Masse, ca. 0,25kg/h, Lösungsmittel. Die benötigte Verdampfungsenthalphie wird dem flüssigen System durch Temperaturabsenkung entzogen. Durch die Verdampfung des Lösungsmittel wird das System an LL übersättigt. Die Übersättigung wird durch gezieltes Kristallwachstums auf der vorhandenen Feststoffoberfläche abgebaut. Die mittlere Verweilzeit der flüssig-festen Phase beträgt ca. 2,5h. Die erzeugte Suspension ca. 1,25kg/h wird absatzweise im 10 min Takt abgenommen.

Die Suspension wird über eine gewöhnliche Laborfilterzentrifuge gegeben. Hierbei wird die Mutterlauge (ca. 0,85kg/h) vom festen feuchten Zielprodukt (ca. 0,4kg/h) dem festen Laurinlactam getrennt. Zur Anhebung der Produktqualität wird der Feststoff mit kaltem (vorzugsweise mit einer Temperatur kleiner 65 °C) Lösungsmittel (ca. 0,4kg/h) gewaschen. Hiermit wird eine Wäsche der Feststoffoberfläche erzielt, wodurch unerwünschte Nebenkomponenten entfernt werden. Der feuchte Feststoff wird im Trockenschrank unter Vakuum (100mbar) bei 90°C getrocknet. Nach erfolgter Trocknung verbleibt ein Feststoff von 0,275kg/h. Dies entspricht einer Ausbeute von 91,7% Laurinlactam.

Die Aufarbeitung der flüssigen Mutterlauge bedarf keine Nachstellung im Labor, da grundsätzliche Verschaltung aus dem "herkömmlichen" Prozess bekannt ist.
Ullmann's Encyclopedia of Industrial Chemistry Cyclododecanol, Cyclododecanone, and Laurolactam Thomas Schiffer and Georg Oenbrink Jahr 2005 Wiley-VCH Verlag GmbH & Co. KGaA, Weinheim 10.1002/14356007.a08_201

### Lösemittelauswahl

Für das aufgezeigte Verfahren wird bevorzugt Hydrocumol als Lösemittel verwendet. Alternativ können aber auch die nachfolgend aufgeführten Lösungsmittel eingesetzt werden. Hierbei können verschiedene Komponenten oder Mischungen von Komponenten zum Einsatz kommen, wie z.B. Hydrocumol (HC), Cyclododecanon (CDON), Cyclododecatrien (CDT), Toluol, Cycloheptan, Cyclooctan (COA), Cyclononan, Cyclodecan, Cyclododcan, Vinylcyclohexan (VCH) oder Ethylcyclohexan (ECH), Dimethycyclohexan, tert.-Butylcyclohexan.

Des Weiteren sind Kombinationen der aufgeführten Lösungsmittel möglich, da hierbei das eutektische Verhalten, also die Schmelzpunkterniedrigung, der Gemische vorteilhaft ausgenutzt werden kann. Eine Absenkung der Schmelztemperatur des Lösungsmediums (Gemisch verschiedener Lösungsmittel) reduziert den apparativen Aufwand, da auf eine aufwendige Begleitheizung verzichtet werden könnte.

## Patentansprüche

1. Verfahren zur Aufarbeitung eines Laurinlactam enthaltenen Stoffstroms für die Rückgewinnung aller enthaltenen Komponenten,
**dadurch gekennzeichnet,**
**dass** der aus der Synthese kommende Stoffstrom, der Laurinlactam und weitere Komponenten enthält, zunächst abgekühlt wird, so dass selektiv durch Lösungskühlungskristallisation nur die Löslichkeitsgrenze von Laurinlactam überschritten und Laurinlactam gezielt auskristallisiert und in einer nachgeschalteten Fest-Flüssig-Trennung von der Mutterlauge abgetrennt wird und die Mutterlauge anschließend einer mehrstufigen Destillationssequenz zugeführt wird.

2. Verfahrens nach Anspruch 1,
**dadurch gekennzeichnet,**
**dass** die Kristallisation als Lösungskristallisation ausgeführt wird.

3. Verfahrens nach Anspruch 2,
**dadurch gekennzeichnet,**
**dass** die Lösungskristallisation als Lösungskühlungskristallisation ausgeführt wird.

4. Verfahrens nach Anspruch 3,
**dadurch gekennzeichnet,**
**dass** die Lösungskühlungskristallisation als Flashkühlungskristallisation ausgeführt wird.

5. Verfahren nach Anspruch 1, 2, 3 oder 4
**dadurch gekennzeichnet,**
**dass** größer 80 % des Laurinlactams bereits bei der Kristallisation abgetrennt werden.

6. Verfahren nach Anspruch 1, 2, 3, 4 oder 5,
**dadurch gekennzeichnet,**
**dass** das bei der Kristallisation abgetrennte Laurinlactam zur Entfernung der anhaftenden Mutterlauge mit einer Waschflüssigkeit gewaschen wird.

7. Verfahren nach einem der vorhergehenden Ansprüche,
**dadurch gekennzeichnet,**
**dass** das bei der Kristallisation abgetrennte Laurinlactam zur Entfernung der anhaftenden Mutterlauge mit Hydrocumol gewaschen wird.

8. Verfahren nach Anspruch 6,
**dadurch gekennzeichnet,**
**dass** als Waschflüssigkeit Hydrocumol (HC), Cyclododecanon (CDON), Cyclododecatrien (CDT), Toluol, Cycloheptan, Cyclooctan (COA), Cyclononan, Cyclodecan, Cyclododcan, Vinylcyclohexan (VCH) oder Ethylcyclohexan (ECH), Dimethycyclohexan, tert.-Butylcyclohexan oder ein Gemisch davon eingesetzt werden kann.

9. Verfahren nach einem der vorhergehenden Ansprüche,
**dadurch gekennzeichnet,**
**dass** das bei der Kristallisation abgetrennte Laurinlactam einem Trocknungsschritt unterzogen wird.

10. Verfahren nach Anspruch 9,
**dadurch gekennzeichnet,**
**dass** das bei der Lösungskühlungskristallisation abgetrennte Laurinlactam bereits eine Reinheit von >99% aufweist.

11. Verfahren nach Anspruch 1,
**dadurch gekennzeichnet,**
**dass** die Destillation vor der Kristallisation durchgeführt wird.

12. Verfahren nach Anspruch 11,
**dadurch gekennzeichnet,**
**dass** der aus der Synthese kommende Stoffstrom, der Laurinlactam und weitere Komponenten enthält, einer destillativen Trennsequenz zugeführt werden, worin die enthaltenen Leichtsieder mit Lösungsmittel über Kopf destilliert werden, der Sumpf der Leichtsiederkolonne, der unter anderem Laurinlactam enthält, einer Lösungskristallisation zugeführt wird.

13. Verfahrens nach Anspruch 12,
**dadurch gekennzeichnet,**
**dass** die Lösungskristallisation als Lösungskühlungskristallisation ausgeführt wird.

14. Verfahrens nach Anspruch 13,
**dadurch gekennzeichnet,**
**dass** die Lösungskühlungskristallisation als Flashkühlungskristallisation ausgeführt wird.

15. Verfahren nach Anspruch 11,
**dadurch gekennzeichnet,**
**dass** der aus der Synthese kommende Stoffstrom, der Laurinlactam und weitere Komponenten enthält, zunächst über einen Wärmetauscher zwecks Temperaturerhöhung geführt wird, dann in einem Flashbehälter entspannt wird, anschließend die Dampfphase einer destillativen Trennsequenz zugeführt wird, die Schwersiedende Fraktion, die unter anderem Laurinlactam enthält zusammen mit der Flashstufe, die den Hauptteil des Laurinlactams enthält, einer Schmelzkristallisation zugeführt wird.
